# NEW EUROPEAN PATENT SPECIFICATION

(11) **EP 3 517 169 B2**
(45) Date of publication and mention of the opposition decision: **28.10.2026**
(45) Mention of the grant of the patent: 08.06.2022
(21) Application number: 19156900.3
(22) Date of filing: 21.04.2011
(51) Int. Cl.: A61N 1/36, A61N 1/37, A61N 1/372

(54) **MRI-SAFE DISK MAGNET FOR IMPLANTS**
MRT-SICHERER PLATTENMAGNET FÜR IMPLANTATE
AIMANT À DISQUE SANS RISQUE SOUS IRM POUR IMPLANTS

(30) Priority: 23.04.2010 US 32715810 P
(43) Date of publication of application: 31.07.2019
(60) Divisional application: 22177545.5 / 4 074 373; 23199801.4; 23206150.7; 23212069.1; 23219919.0; 24153265.4; 24159141.1; 24165616.4; 24171701.6; 24177408.2; 24182993.6; 24189649.7; 24194843.9; 24200516.3; 24206955.7; 24213346.0; 24219868.7; 25151544.1; 25157781.3; 25163553.8; 25170428.4; 25176425.4; 25182648.3; 25189139.6; 25194502.8; 25200069.0; 25206444.9; 25212782.4; 25219354.5; 25226967.5; 26153463.0; 26160128.0; 26166605.1; 26174125.0
(62) Document number(s) of the earlier application(s) in accordance with Art. 76 EPC: 16189856.4 / 3 138 605; 11717431.8 / 2 560 730
(73) Proprietor: MED-EL Elektromedizinische Geräte GmbH, 6020 Innsbruck (AT)
(72) Inventor: ZIMMERLING, Martin, 6082 Patsch (AT); JAMNIG, Bernhard, 6020 Innsbruck (AT)
(74) Representative: Lucke, Andreas

(56) References cited:
- WO-A1-2008/109800
- WO-A1-2010/042463
- WO-A2-03/081976
- US-A1- 2009 299 437

## Description

### FIELD OF THE INVENTION

The present invention relates to implantable medical devices, and specifically, to magnetic elements in such devices that allow for magnetic resonance imaging.

### BACKGROUND ART

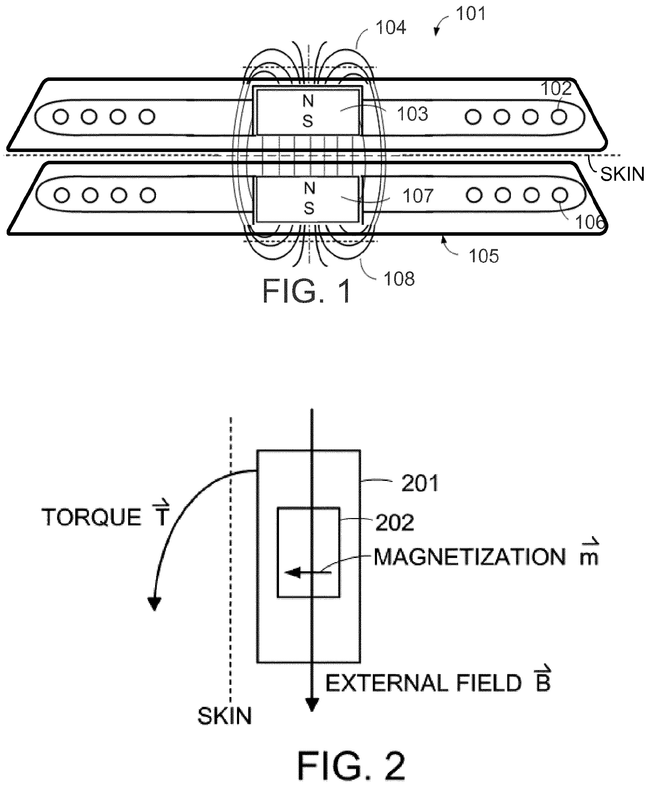

Some hearing implants such as Middle Ear Implants (MEI's) and Cochlear Implants (CI's) employ attachment magnets in the implantable part and an external part to hold the external part magnetically in place over the implant. For example, as shown in Fig. 1, a typical cochlear implant system may include an external transmitter housing **101** containing transmitting coils **102** and an external magnet **103.** The external magnet **103** has a conventional coin-shape and a north-south magnetic dipole that is perpendicular to the skin of the patient to produce external magnetic field lines **104** as shown. Implanted under the patient's skin is a corresponding receiver assembly **105** having similar receiving coils **106** and an implanted internal magnet **107.** The internal magnet **107** also has a coin-shape and a north-south magnetic dipole that is perpendicular to the skin of the patient to produce internal magnetic field lines **108** as shown. The internal receiver housing **105** is surgically implanted and fixed in place within the patient's body. The external transmitter housing **101** is placed in proper position over the skin covering the internal receiver assembly **105** and held in place by interaction between the internal magnetic field lines **108** and the external magnetic field lines **104.** Rf signals from the transmitter coils **102** couple data and/or power to the receiving **coil 106** which is in communication with an implanted processor module (not shown).

One problem arises when the patient undergoes Magnetic Resonance Imaging (MRI) examination. Interactions occur between the implant magnet and the applied external magnetic field for the MRI. As shown in Fig. 2, the direction magnetization ${m}^{⇀}$ of the implant magnet **202** is essentially perpendicular to the skin of the patient. Thus, the external magnetic field ${B}^{⇀}$ from the MRI may create a torque ${T}^{⇀}$ on the internal magnet **202,** which may displace the internal magnet **202** or the whole implant housing **201** out of proper position. Among other things, this may damage the adjacent tissue in the patient. In addition, the external magnetic field ${B}^{⇀}$ from the MRI may reduce or remove the magnetization ${m}^{⇀}$ of the implant magnet **202** so that it may no longer be strong enough to hold the external transmitter housing in proper position. The implant magnet **202** may also cause imaging artifacts in the MRI image, there may be induced voltages in the receiving coil, and hearing artifacts due to the interaction of the external magnetic field ${B}^{⇀}$ of the MRI with the implanted device. This is especially an issue with MRI field strengths exceeding 1.5 Tesla.

Thus, for existing implant systems with magnet arrangements, it is common to either not permit MRI or at most limit use of MRI to lower field strengths. Other existing solutions include use of a surgically removable magnets, spherical implant magnets (e.g. U.S. Patent 7,566,296), and various ring magnet designs (e.g., U.S. Provisional Patent 61/227,632, filed July 22, 2009). Among those solutions that do not require surgery to remove the magnet, the spherical magnet design may be the most convenient and safest option for MRI removal even at very high field strengths. But the spherical magnet arrangement requires a relatively large magnet much larger than the thickness of the other components of the implant, thereby increasing the volume occupied by the implant. This in turn can create its own problems. For example, some systems, such as cochlear implants, are implanted between the skin and underlying bone. The "spherical bump" of the magnet housing therefore requires preparing a recess into the underlying bone. This is an additional step during implantation in such applications which can be very challenging or even impossible in case of very young children.

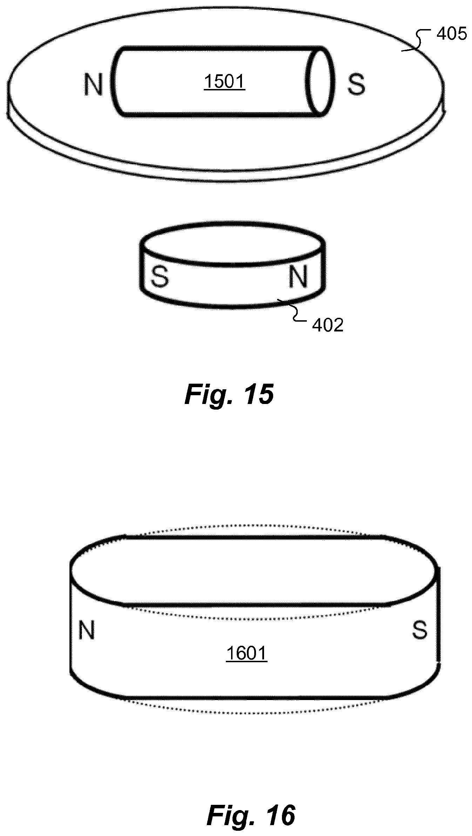

Such an implantable system with a spherical magnet is known from WO 03/081976 A2, in which an implantable system for a recipient patient is disclosed, comprising an implantable coil housing containing a signal coil for transcutaneous communication of an implant communication signal. The implant coil housing comprises a spherical first attachment magnet for transcutaneous magnetic interaction with a corresponding second attachment magnet. The spherical first attachment magnet is free to rotate such that it is capable of aligning with an external magnetic field. In the embodiment shown in Fig. 15 of this prior art document, the coil housing has a non-flat bottom facing the underlying bone with a "spherical bump" of the aforementioned type accommodating the spherical magnet, which as mentioned above would require drilling a recess into the bone underlying the coil housing during implantation.

### SUMMARY OF THE INVENTION

The invention is defined in the appended claims. Aspects, embodiments and examples disclosed herein which do not fall within the scope of the appended claims do not form part of the invention, and are merely provided for illustrative purposes.

Embodiments of the present invention are directed to a magnetic arrangement for an implantable system for a recipient patient. A planar coil housing contains a signal coil for transcutaneous communication of an implant communication signal. A first attachment magnet is located within the plane of the coil housing and rotatable therein (e.g., a planar disk shape), and has a magnetic dipole parallel to the plane of the coil housing for transcutaneous magnetic interaction with a corresponding second attachment magnet.

Further specific embodiments may also have at least one magnetic focus director within the housing adjacent to the first attachment magnet and transcutaneously directing the magnetic field to increase magnetic attraction force between the first and second attachment magnets by focusing the magnetic flux (i.e. locally increasing magnetic induction). The focus director may also be used to guide magnetic field lines away from magnetically sensitive components such as implanted sensors or ferrite-based components.

The coil housing may be an implant coil housing for implantation under the skin of the patient and the signal coil would then be a receiver coil. There may also be an implant signal processor within the housing for processing the implant communication signal, and a magnetic switch within the coil housing and magnetically interacting with the first attachment magnet so as to affect operation of the signal processor as a function of magnetic orientation of the first attachment magnet. Or the coil housing may be an external coil housing for placement on the skin of the patient and the signal coil would then be a transmitter coil.

The first attachment magnet may be adapted to rotate within the coil housing in response to an external magnetic field, and there may be a lubrication coating covering at least a portion of the first attachment magnet and reducing friction between the first attachment magnet and the coil housing to promote the rotation of the first attachment magnet. At least one of the attachment magnets may have a planar disc shape, a rectangular beam shape, a cylindrical beam shape, or a cut away disc shape. Or at least one of the attachment magnets may comprise a pair of complementary cylindrical attachment magnets, which optionally may further include a magnetic flux guide connecting the pair of complementary cylindrical attachment magnets.

In any of the above, the implantable system may be a cochlear implant system, a middle ear implant system, a vestibular implant system, or a laryngeal pacemaker implant system.

### BRIEF DESCRIPTION OF THE DRAWINGS

Figure 1 shows portions of a typical cochlear implant system.
Figure 2 illustrates the interactions that can occur between an implant magnet and the applied external magnetic field for an MRI system.
Figure 3A-B compares the perpendicular magnetic dipole arrangement in typical existing implant attachment magnets with the parallel magnetic dipole arrangement in an attachment magnet according to an embodiment of the present invention.
Figure 4A-B show elevated perspective and side cross-sectional views of a cochlear implant coil housing having an attachment magnet according to an embodiment of the present invention.
Figure 5 shows a patient in a typical closed MR scanner where the main magnetic field goes from head to toe on the patient.
Figure 6 shows a patient in a typical open MR scanner where the main magnetic field goes from front to back on the patient.
Figure 7 A-B shows the case where the magnetization direction of the attachment magnet is not perpendicular to the main external MRI field.
Figure 8 A-B shows side and top view structural details of an embodiment having a soft magnetic focus director arrangement.
Figure 9 shows an embodiment having a magnetic switching arrangement having a switching operation which is a function of the orientation of the attachment magnet.
Figure 10 shows an embodiment similar to the one in Fig. 8 which includes use of a horseshoe shaped magnet.
Figure 11 shows a side view of an embodiment having two corresponding cylindrical external attachment magnets.
Figure 12 shows an elevated perspective view of some of the same structures as in Fig. 11.
Figure 13 shows a similar arrangement with an additional magnetic flux guide.
Figure 14 shows an embodiment having an external attachment magnet with a rectangular beam shape.
Figure 15 shows an embodiment having an external attachment magnet with a cylindrical beam shape.
Figure 16 shows an embodiment having an external attachment magnet based on a disc shape with the less magnetic regions cut away.

### DETAILED DESCRIPTION OF SPECIFIC EMBODIMENTS

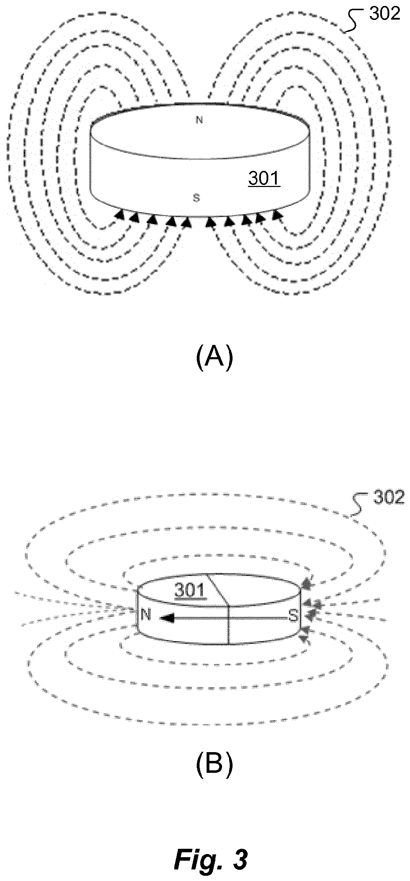

Various embodiments of the present invention are directed to a magnetic arrangement for an implantable system for a recipient patient which is compatible with MRI systems. Figure 3A shows the magnetic field arrangement in typical existing implant attachment magnets. In this case, the attachment magnet **301** is disk-shaped (i.e., cylindrical) with the north-south magnetic dipole realized in the axial direction as is conventional producing magnetic field lines **302** as shown. Embodiments of the present invention change the direction of magnetization as shown in Figure 3B so that the north-south magnetic dipole is oriented across the diameter of the attachment magnet **301** parallel to (i.e., "in") the plane of the coil housing, producing magnetic field lines **302** as shown.

Of course, with such an arrangement, it is important that both the internal implant receiver attachment magnet and the external transmitter attachment magnet be magnetized with the same orientation in the plane of the coil housing (i.e., parallel to the skin). Then when the external coil housing is placed onto the patient's skin over the implant coil housing, the two attachment magnets turns around on their axis such that the north and south poles of one attachment magnet are positioned adjacent to south and north poles respectively of the other attachment magnet thereby maximizing the attractive magnetic force between the two.

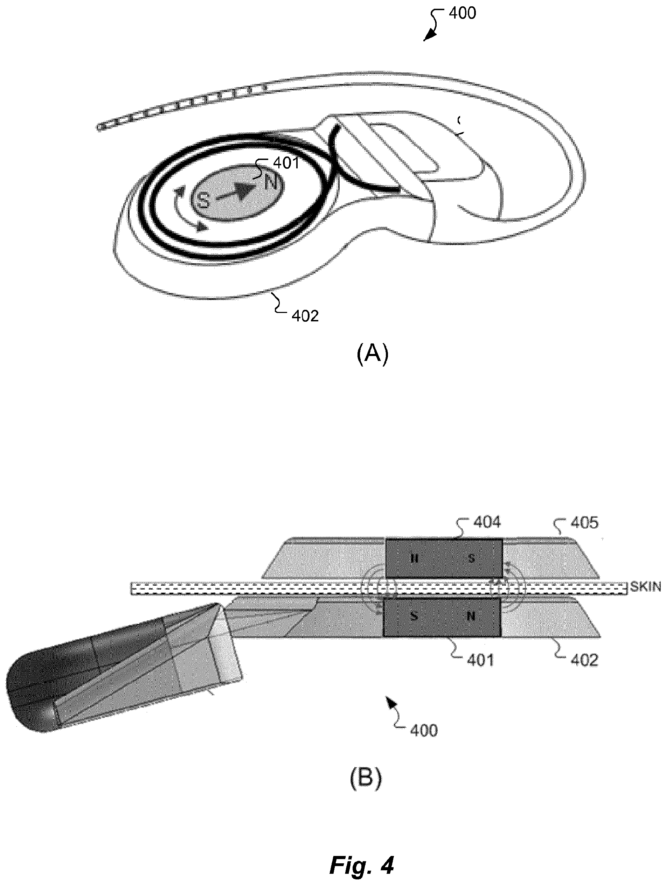

Figure 4A shows an elevated perspective view and Figure 4B shows a side cross-sectional view of a cochlear implant **400** having a planar coil housing **402** that contains a signal coil for transcutaneous communication of an implant communication signal. A first attachment magnet **401** is located within the plane of the coil housing **402** and rotatable therein (e.g., a planar disk shape) has a magnetization direction with a magnetic dipole parallel to the plane of the coil housing **402.** An external transmitter coil housing **405** with a corresponding second attachment magnet **404** with a similar magnetic dipole direction parallel to the plane of its coil housing **405** so that when placed on the skin of the recipient patient, their respective magnetic fields cause the two attachment magnets **401** and **404** to self-orient as described above to form a magnetic attraction connection between them. In specific embodiments, the coil housing **402** may be made have a titanium case with the attachment magnet **401** located outside the titanium case, for example, embedded in a silicone coil assembly. Alternatively, the coil housing **402** may be a ceramic case where the attachment magnet **401** is hermetically encapsulated within the ceramic housing.

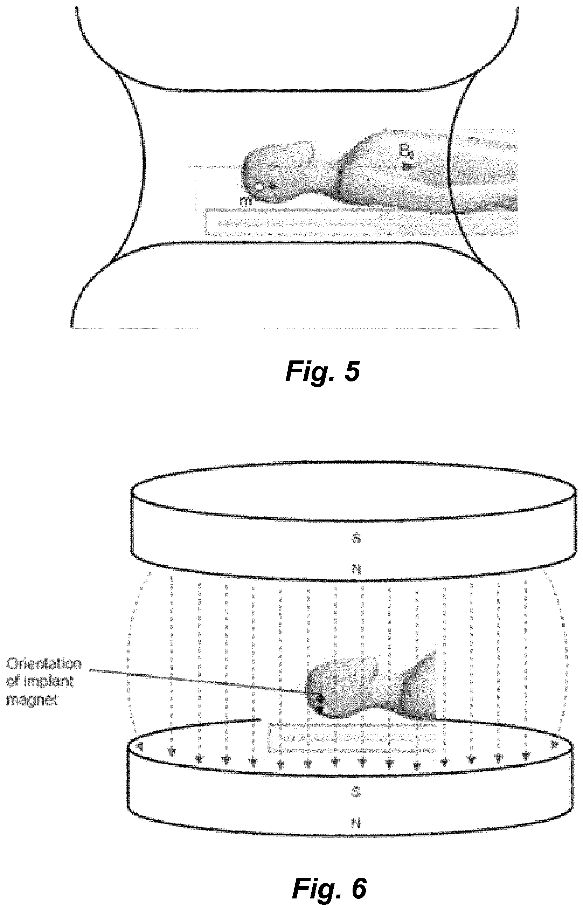

When a person wearing an implant with such an attachment magnet needs to undergo an MRI, they can enter the scanner room after the external components of the implant system have been removed. As the implant user is brought into the MR scanner, the attachment magnet may have a component of its magnetization which is perpendicular to the external magnetic field of the MR scanner. This will result in the attachment magnet turning around on its axis to align the magnetization direction of its magnetic dipole with the static field of the MR scanner. This occurs in both conventional closed MR scanners characterized by a bore with a horizontal static magnetic field as shown in Figure 5 running parallel to the body axis from head to toe on the patient, as well as in so-called open MR scanners as shown in Figure 6 characterized by a vertical static magnetic field running perpendicular to the body axis through the body of the patient from front to back.

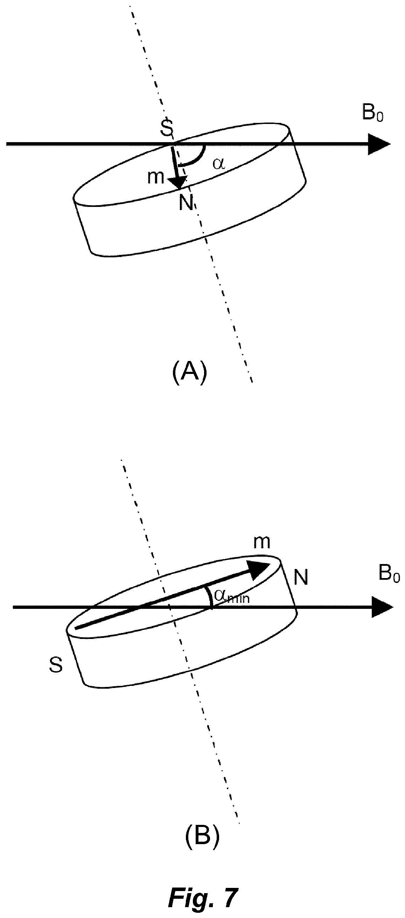

When the attachment magnet of the implant can align with the static magnetic field of the MR system, there is no torque exerted by the static magnetic field of the MR on the attachment magnet/coil housing arrangement, nor is the magnetic force of the attachment magnet weakened. This is also the case when the attachment magnet cannot align completely with the static magnetic field of the MR scanner, but remains at an angle up to about 20° between the magnetic momentum of the implant magnet and the static magnetic field of the MR scanner. Since the torque is proportional to the sine of the angle, for example, the torque is reduced for a remaining angle of 20° to about 1/3 (66% reduction) of the torque when the attachment magnet remains fixed at the worst case angle of 90°. In situations where the rotational axis of the attachment magnet (i.e., its axis of symmetry) is exactly perpendicular to the static magnetic field of the MR system, the attachment magnet can turn around and can align its magnetic dipole m exactly with the static magnetic field *B₀* without torque or demagnetization. But this is an ideal theoretical case, in most real situations the rotational axis of the attachment magnet is just close to but not exactly perpendicular to the static magnetic field, e.g., at an angle of 70° or 80° instead of 90°. This is shown in Fig. 7 A. The attachment magnet will turn around on its axis and will try to align its magnetic dipole *m* with the surrounding magnetic field *B₀* as best possible (Fig. 7B). There will remain a small angle (α₂), and the residual torque is proportional to the sine of this angle (e.g. torque is only about 1/3 when the remaining angle is of 20°). Since the remaining angle typically is small (<<90°) there is virtually no risk for an attachment magnet weakening even for high values of *B₀* (> 1.5 Tesla). It will only be when (or if) the rotational axis of the attachment magnet is (almost) parallel to the static magnetic field *B₀* of the MR system (as in the prior art) that the attachment magnet would then have a magnetic dipole which is essentially perpendicular to *B₀* (regardless of how the magnet turns around), and the full torque and risk of magnet weakening would be present.

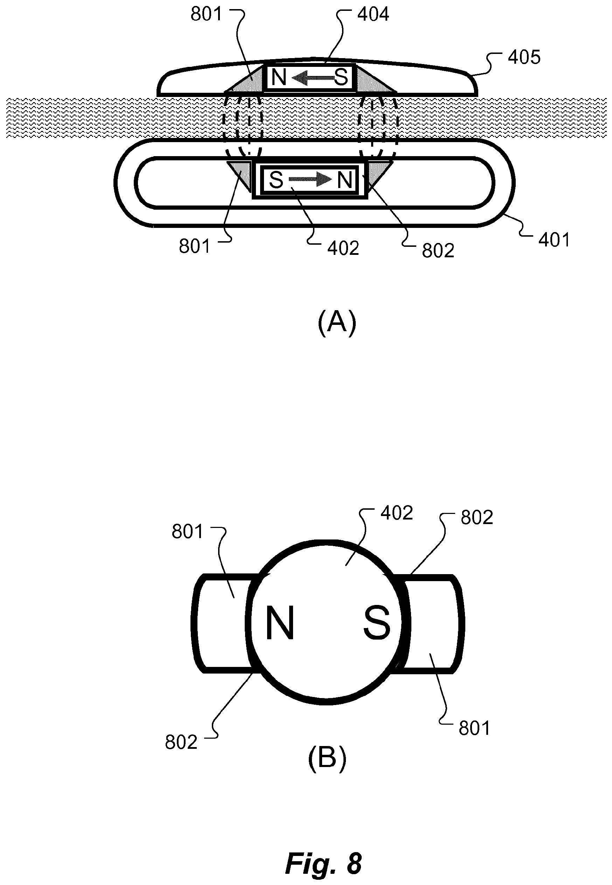

Figure 8 A-B shows side and top view structural details of an embodiment wherein the coil housing **401** also contains a magnetic focus director **801** which surrounds some or all of the attachment magnet **402.** The magnetic focus director **801** is made of a soft ferromagnetic material that directs the magnetic field of the attachment magnet **402** through the skin to increase the magnetic attraction force with the other corresponding attachment magnet by focusing the magnetic flux (i.e. locally increasing magnetic induction). The focus director **801** may also be used to guide magnetic field lines away from magnetically sensitive components such as implanted sensors or ferrite-based components. In the specific embodiment shown in Fig. 8 A-B, the magnetic focus director **801** has two generally rectangular opposing director pieces located on opposite sides of the attachment magnet **402** which then may have a defined equilibrium position in the absence of an external magnetic field such as an MRI field. The embodiment shown in Fig. 8 also shows a lubrication coating **802** made of polytetrafluoroethylene (PTFE) that covers at least a portion of the attachment magnet **402** and reduces the friction between the attachment magnet **402** and the coil housing **401** so as to promote the rotation of the attachment magnet **402** in response to external magnetic fields.

In some embodiments, the attachment magnet may be fixed within the external component (e.g., transmitter coil housing) to prevent its rotation. For example, the external attachment magnet may be fixed within the external component so that its magnetic axis is in a well-defined orientation when the external component is worn on the body. The position of the external component can then adjusted for the best magnet orientation to achieve the optimal (maximum) magnetic fixation of the external component.

Alternatively, the attachment magnet may be encapsulated within the external component so that it can rotate on its axis like the attachment magnet in the implant. In some hybrid embodiments, the implant attachment magnet may be not completely free to turn around, but may be limited to a certain maximum rotation angle. When both the internal and the external attachment magnets are free to rotate, if at least one of the attachment magnets has its center of gravity offset from its rotational axis, then that magnet will turn around when the implant user turns around relative to the magnet axis. Since both attachment magnets are magnetically coupled, the implant attachment magnet will also turn around. Based on this arrangement, a (one-axis) gyro sensor could be implemented, for example, for an electronic vestibular prosthesis. In a different embodiment, the implant attachment magnet may have a restoring force which positions it into a defined orientation as long as no external magnetic field is present.

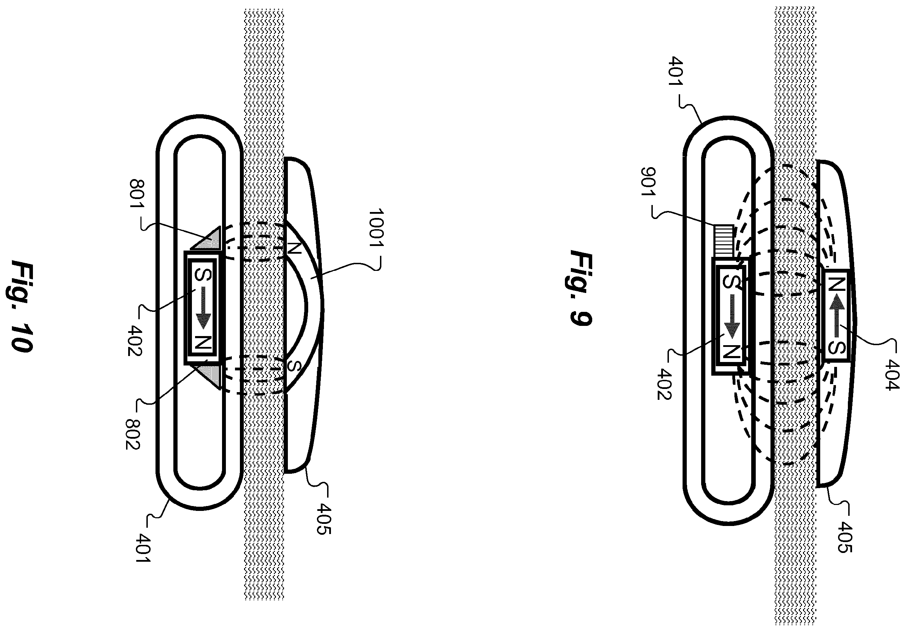

Figure 9 shows an embodiment which includes a magnetic switch **901** within the coil housing **401.** The magnetic switch **901** magnetically interacts with the attachment magnet **402** so as to affect the operation of the implant signal processor as a function of the orientation of the attachment magnet **402.** For example, with the south pole of the attachment magnet **402** facing downwards (caudally) indicates that an external transmitter coil is located over the coil housing **401** so the implant signal processor is activated. On the other hand, reorienting the attachment magnet **402** so that the north magnetic pole faces downwards could trigger a different operating mode, e.g., a telemetry mode, recharging or programming mode, or for activating / deactivating electrodes. Such functionality would require that the external attachment magnet be fixed within its housing to prevent its rotation.

It is worth noting that while the embodiments described above are disk shaped (cylindrical), but that is not necessarily required. Rather, any shape could be implemented so long as the magnetization is parallel to the coil housing and the skin. For example, Figure 10 shows an embodiment similar to the one in Fig. 8 which includes use of a horseshoe shaped attachment magnet **1001.** In addition, in the embodiments described above, the attachment magnets have a magnetization axis that perpendicular to the rotational axis of the disk, but in other embodiments, the attachment magnets may more generally have a magnetization axis that is not parallel to the rotational axis.

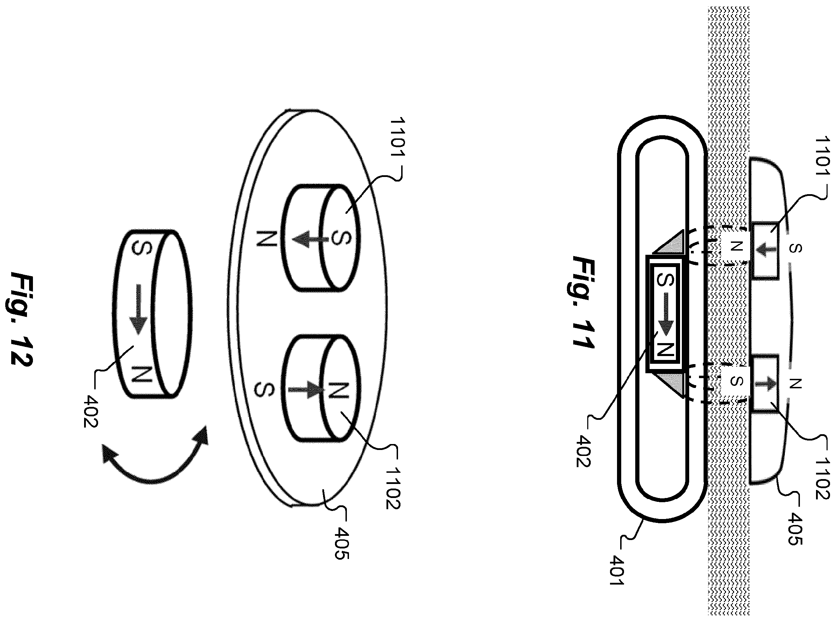

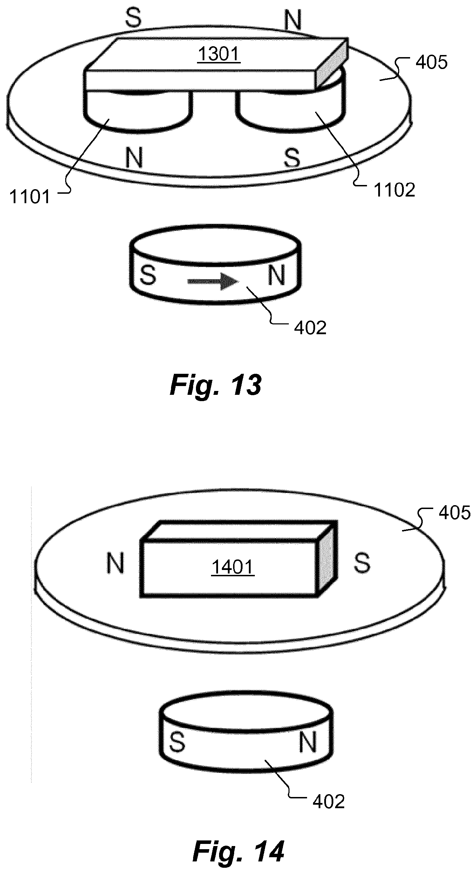

Figure 11 shows a side view of another embodiment wherein the external coil housing **405** contains a pair of complementary cylindrical attachment magnets **1101** and **1102** with opposite magnetic polarities as shown which interact with the single cylindrical implant attachment magnet **402** which is free to rotate in the plane of the implant housing **405** to orient itself to magnetically interact with the external attachment magnets **1101** and **1102.** Fig. 12 shows an elevated perspective view of some of the same structures. Fig. 13 shows a similar arrangement with an additional magnetic flux guide **1301** connecting the two external attachment magnets **1101** and **1102.**

Figure 14 shows an embodiment having an external attachment magnet **1401** with a rectangular beam shape. Figure 15 shows an embodiment having an external attachment magnet **1501** with a cylindrical beam shape. Figure 16 shows an embodiment having an external attachment magnet **1601** based on a disc shape with the less magnetic regions cut away to save weight and volume without significantly compromising overall magnetic field strength.

Optimizing the external attachment magnet arrangement minimizes the total mass and controls the spatial distribution of the magnetic field, which in turn can influence the electronic circuitry related to the external coil-e.g., reduced influence of the external attachment magnets on the inductive signal transmission properties. In addition, properly optimized design of the external attachment magnet can offer improved magnetic field distance characteristics, e.g., allowing a shallower field. Some embodiments may have similar implant attachment magnet arrangements.

Non-spherical shaped magnets with a magnet field oriented in the plane of the coil housing (i.e., parallel to the skin) basically the same advantages with regards to MR systems as with spherical magnet designs, with the main limitation being that the diskshape attachment magnet design described above allows for rotation of the magnet in only one plane. Still when the implant is placed inside the body in a sagittal plane orientation (as with a hearing implant) and with a standard MRI examination position of the patient (i.e. in supine position with the head kept straight), the implant attachment magnet can align quite well with the static magnetic field both in closed MR scanners (with a horizontal main magnetic field) as well as is open MR scanners (with the main magnetic field in vertical direction).

Attachment magnets according to embodiments of the present invention present a slim profile which is safe for MRI field strengths up to and beyond 3 Tesla without the need to surgically remove the implant magnet. Alternatively, in some embodiments the implant attachment magnet may be adapted to be temporarily removable by minor surgery from the implant coil housing if desired to reduce MRI artifacts.

In contrast to spherical design attachment magnets, the present coil housing can have a flat bottom so that there is no need to drill a recess into the bone during implantation of the device. This makes such a magnet design especially well-suited for implantation in young children. Moreover, embodiments can be equally effective where there is a relatively large magnet in the implanted part and a relatively small magnet in the external part, and vice versa. And due to the different magnetization direction, it is expected that the MR imaging artifact may be smaller compared to conventional implant magnets, for example, extending less in the medial direction.

Compared to the conventional disk magnet concept with axial magnetization, embodiments of the present invention have attractive forces on both poles, and the attraction is caused by two forces which apply at the two poles of each magnet. The result is that the shear force between the external attachment magnet and the implant attachment magnet is higher in the direction of the magnetization axis of the two magnets. By turning the external attachment magnet for optimal orientation over the implant (e.g. vertical magnetic axis), a better magnetic attachment of the external parts can be achieved. In such an arrangement, the external attachment magnet also stays in place over the implant attachment magnet with less lateral displacement even in response to small mechanical shocks. The present embodiments also have a better (shallower) force-over-distance diagram than two conventional magnets with axial magnetization. It may be advantageous if the attractive force does not vary greatly over the distance between the two attachment magnets.

With standard supine patient position where the implant attachment magnet is oriented in a coronal plane, embodiments of the attachment magnet described here can align well with the static magnetic field in closed MR scanners only while such an implant magnet in axial orientation would only align with the static magnetic field in open scanners with vertical magnetic field. The torque exerted to the implant can remain relatively high when the implant magnet which has only one degree of freedom cannot align well enough with the external magnetic field.

## Claims

1. An implantable system for a recipient patient, said implantable system being one of a cochlear implant system, a middle ear implant system, or a vestibular implant system, and comprising a magnetic arrangement, the arrangement comprising:
a planar coil housing (402) containing a signal coil for transcutaneous communication of an implant communication signal;
a first attachment magnet (401) within the plane of the coil housing, rotatable only in said plane of the coil housing, and having a magnetic dipole parallel to the plane of the coil housing for transcutaneous magnetic interaction with a corresponding second attachment magnet (404), wherein the coil housing is an implant coil housing (402) for implantation under the skin of the patient and wherein the signal coil is a receiver coil,
wherein said implant coil housing (402) has a flat bottom so that there is no need to drill a recess into the bone during implantation,
wherein said first attachment magnet (401) has a non-spherical shape allowing said implant coil housing to have said flat bottom.

## Patentansprüche

1. Implantierbares System für einen Empfängerpatienten, wobei das implantierbare System ein Cochleaimplantatsystem, ein Mittelohrimplantatsystem oder ein Vestibularimplantatsystem ist und eine magnetische Anordnung umfasst, wobei die Anordnung Folgendes umfasst:
ein ebenes Spulengehäuse (402), welches eine Signalspule zur transkutanen Kommunikation eines Implantat-Kommunikationssignals enthält;
einen ersten Befestigungsmagneten (401) innerhalb der Ebene des Spulengehäuses, der nur in der Ebene des Spulengehäuses drehbar ist, und einen zu der Ebene des Spulengehäuses parallelen magnetischen Dipol zur transkutanen magnetischen Wechselwirkung mit einem zugehörigen zweiten Befestigungsmagneten (404) aufweist, wobei das Spulengehäuse ein Implantat-Spulengehäuse (402) zur Implantation unter die Haut eines Patienten ist und wobei die Signalspule eine Empfängerspule ist,
wobei das Implantatspulengehäuse (402) einen flachen Boden hat, sodass es nicht notwendig ist, während der Implantation eine Aussparung in den Knochen zu bohren,
wobei der erste Befestigungsmagnet (401) ein Magnet mit einer nicht-sphärischen Form ist, der ein schlankes Profil bietet, welches gestattet, dass das Implantatspulengehäuse den genannten flachen Boden hat.

## Revendications

1. Système implantable pour un patient receveur, ledit système implantable étant l'un d'un système d'implant cochléaire, d'un système d'implant d'oreille moyenne ou d'un système d'implant vestibulaire comprenant un agencement magnétique, l'agencement comprenant :
un boîtier de bobine plan (402) contenant une bobine de signal pour une communication transcutanée d'un signal de communication d'implant ;
un premier aimant de fixation (401) à l'intérieur du plan du boîtier de bobine, ne pouvant tourner qu'à l'intérieur dudit plan du boîtier de bobine, et présentant un dipôle magnétique parallèle au plan du boîtier de bobine pour une interaction magnétique transcutanée avec un second aimant de fixation (404) correspondant, dans lequel le boîtier de bobine est un boîtier de bobine d'implant (402) destiné à être implanté sous la peau du patient et dans lequel la bobine de signal est une bobine réceptrice,
dans lequel ledit boîtier de bobine d'implant (402) présente un fond plat de sorte qu'il n'est pas nécessaire de percer un évidement dans l'os lors de l'implantation,
dans lequel ledit premier aimant de fixation (401) est un aimant de forme non-sphérique présentant un profil mince permettant audit boîtier de bobine d'implant d'avoir ledit fond plat.
